Europäisches Patentamt

European Patent Office

Office européen des brevets

(11) Veröffentlichungsnummer: **0 256 202**

**A2**

## EUROPÄISCHE PATENTANMELDUNG

(21) Anmeldenummer: **87101802.4**

(51) Int. Cl.4: **G10K 11/00**

(22) Anmeldetag: **10.02.87**

(30) Priorität: **18.08.86 DE 3627943**

(43) Veröffentlichungstag der Anmeldung:
**24.02.88 Patentblatt 88/08**

(84) Benannte Vertragsstaaten:
**DE FR GB NL**

(71) Anmelder: **Siemens Aktiengesellschaft Berlin und München**
**Wittelsbacherplatz 2**
**D-8000 München 2(DE)**

(72) Erfinder: **Reichenberger, Helmut, Dr.**
**Begonienstrasse 28**
**D-8501 Eckental(DE)**

(54) **Ankoppelkörper für eine Stosswellen-Therapieeinrichtung.**

(57) Eine Stoßwellenquelle (1) umfaßt eine Auskoppelmembran (7), die über den Ankoppelkörper (11) z. B. an einen Patienten (13) angekoppelt werden kann. Der Ankoppelkörper (11) besteht aus einem elastischen, formstabilen Material (z. B. einem festen Hydrogel) mit feuchten Außenflächen. Im Ankoppelkörper (11) ist ein Stoßwellensensor (15) enthalten. Der Stoßwellensensor (15) kann dabei als kleiner elektrischer Drucksensor, z. B. piezoelektrischer Sensor mit PVDF-Folie, oder als (bevorzugt optischer) Indikator, beispielsweise in Form einer dünnen Bleifolie, ausgebildet sein. Vorteil dieser Integration von Sensor (15) und Ankoppelkörper (11) ist es, daß während der Behandlung des Patienten (13) die Funktion der Stoßwellenquelle (1) überwacht werden kann. Nach Wartungs-oder Reparaturarbeiten ermöglicht der Einsatz des Ankoppelkörpers (11) eine einfache, korrekte Justierung der Stoßwellenquelle (1) auf ihre ursprünglichen Betriebsdaten (z. B. Position).

FIG 1

EP 0 256 202 A2

## Ankoppelkörper für eine Stoßwellen-Therapieeinrichtung

Die Erfindung betrifft einen Ankoppelkörper für die Ankopplung einer Stoßwelle, insbesondere für die Übertragung von Stoßwellen von einer Stoßwellenquelle zu einem zu behandelnden Patienten, wobei der Ankoppelkörper aus einem elastischen, formstabilen Material mit feuchten Außenflächen gebildet ist.

Im Betrieb einer Stoßwellenquelle, z. B. eines Lithotripters zur Nierensteinzertrümmerung, bei welchem ein Stoßwellenimpuls mit Hilfe einer elektrischen Spule erzeugt wird (vgl. DE-OS 33 28 051), sind von Zeit zu Zeit Überprüfungen der Funktion angebracht. Solche Überprüfungen betreffen beispielsweise die Fokuslage, die Druckverteilung oder die Druckamplitude des Stoßwellenimpulses. Solche Überprüfungen sind regelmäßig im Einsatz der Stoßwellenquelle zweckmäßig; sie sind aber auch notwendig bei Erstmontage, nach Umbauten, beim Service oder bei Reparatur. Wird beispielsweise das den Stoßwellenimpuls fokussierende Mittel (wie z. B. eine akustische Linse oder ein Reflektor) ausgetauscht, so muß hinterher geprüft werden, ob eine identische Fokuslage im Vergleich zur Situation vor dem Austausch vorhanden ist.

Ein Stoßwellensensor, der insbesondere für die Lithotripsie verwendet werden kann, ist aus der DE-OS 34 37 976 bekannt.

Die Erfindung beruht auf der Überlegung, daß als Prüfmittel zur Funktionsüberprüfung sowohl Stoßwellensensoren, insbesondere elektrische Druckmeßelemente, als auch Stoßwellenindikatoren infrage kommen. Bei einem Stoßwellenindikator sollte neben der unmittelbaren Beobachtung des Auftreffpunktes der Stoßwellenimpulse eine nachträgliche Auswertung, z. B. Abschätzung, der integral empfangenen Energie möglich sein. Neben der Herstellbarkeit und dem Preis für die Überprüfung ist die Handhabbarkeit des Prüfmittels von Bedeutung. Auch ist eine in definierter Geometrie möglichst reproduzierbare und verlustfreie Ankopplung wichtig.

Aufgabe vorliegender Erfindung ist es, einen Ankoppelkörper der eingangs genannten Art so auszubilden, daß nach der Ankopplung eine einfache Überprüfung der Funktion der Stoßwellenquelle möglich ist. Insbesondere soll es während des Normalbetriebs der Stoßwellenquelle, also beispielsweise während einer Lithotripsiebehandlung, möglich sein, die Funktion der Stoßwellenquelle zu überwachen.

Diese Aufgabe wird erfindungsgemäß dadurch gelöst, daß im elastischen, formstabilen Material ein Stoßwellensensor enthalten ist.

Der Stoßwellensensor, der vorzugsweise als elektrisches Druckmeßelement aber auch als ein Stoßwellenindikator ausgeführt sein kann, wird bevorzugt in ein formstabiles Hydrogel eingebettet. Als Stoßwellensensoren kommen prinzipiell alle zur Messung einer Stoßwelle geeigneten Prüfmittel infrage, insbesondere aber kleine elektrische Drucksensoren und kleine optische Indikatoren. Der Ankoppelkörper besitzt eine geeignete Form, wie z. B. Scheiben-oder Klotzform, und kann mittels einer Halterung in definierter Relation zur Stoßwellenquelle auf deren Auskoppelfläche aufgesetzt werden. Dabei ergibt sich als Vorteil eine gute Ankopplung des Stoßwellenimpulses an - den Stoßwellensensor. Die Handhabung bei der Überprüfung der Lithotripterfunktion besteht im wesentlichen aus dem Anfeuchten einer Seite des Ankoppelkörpers, dem Befestigen des Ankoppelkörpers an die Stoßwellenquelle und dem Meßvorgang.

Das vorzugsweise verwendete durchsichtige Hydrogel ermöglicht eine direkte Betrachtung oder sogar eine optische Erfassung und Auswertung von Vorder-und/oder Rückseite eines als Sensor eingesetzten Stoßwellenindikators ohne Demontage. Bei Einsatz einer piezoelektrischen aktivierten PVDF-Folie als Stoßwellensensor werden Artefakte, die durch ungewünschte Bewegung der Meßfolie hervorgerufen werden, reduziert.

Weitere Vorteile und Ausgestaltungen der Erfindung ergeben sich aus der folgenden Beschreibung von Ausführungsbeispielen in Verbindung mit den Unteransprüchen. Es zeigen:

Fig. 1 eine Stoßwellenquelle und einen Ankoppelkörper mit integriertem Piezokristall,

Fig. 2 eine Stoßwellenquelle und einen Ankoppelkörper mit eingebauter PVDF-Folie bei kapazitiver Ableitung des Meßsignals,

Fig. 3 einen Ankoppelkörper mit eingebauter PVDF-Folie bei galvanischer Ableitung des Meßsignals und

Fig. 4 eine Stoßwellenquelle und einen Ankoppelkörper mit eingebautem optischen Stoßwellenindikator.

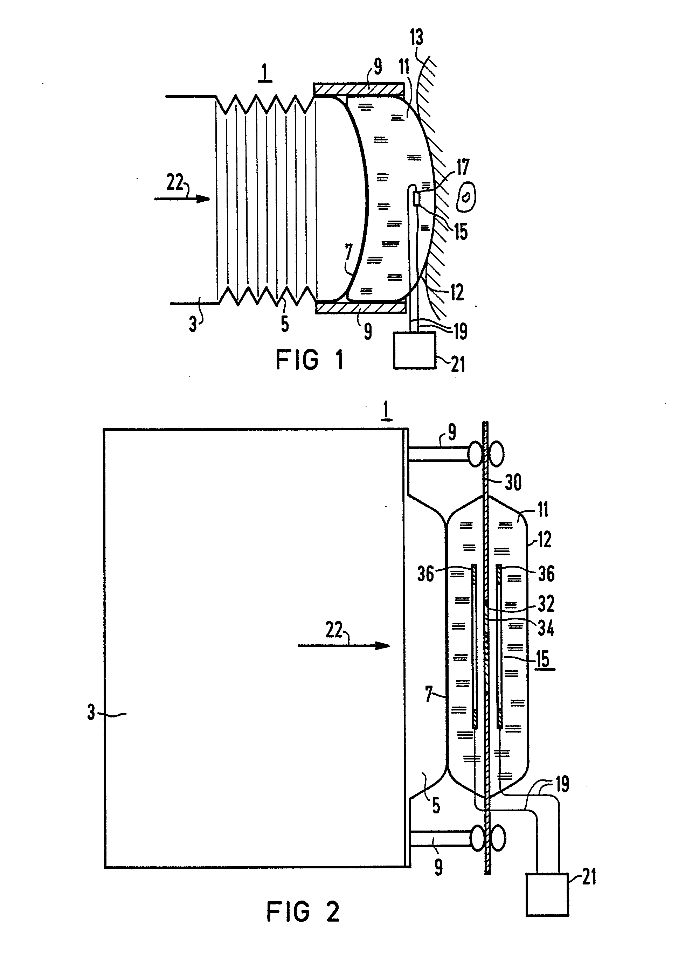

In Fig. 1 ist eine Stoßwellenquelle 1 mit ihren wesentlichen Elementen, nämlich mit einem Stoßwellengenerator 3, einer Vorlaufstrecke 5 mit Fokussierungsmittel und einer Auskoppelmembran 7, dargestellt. Von einer hohlzylindrischen Halterung 9 gestützt, ist an die Auskoppelmembran 7 ein Ankoppelkörper 11 angelegt, der aus einem elastischen, formstabilen Material besteht, insbesondere aus einem Hydrogel mit feuchten Oberflächen. An der freien Stirnseite oder Ankoppelfläche 12 des konkav-konvexen Ankoppelkörpers 11 ist ein Patient 13 angekoppelt. Der Ankoppelkörper 11 dient zur Übertragung von Stoßwellenimpulsen von der Stoßwellenquelle 1 zum Patienten 13. Diese Anordnung ist in der deutschen Patentanmeldung P 36 05 277 (= VPA 86 P 3062) detailliert beschrieben.

In dem Ankoppelkörper 11 ist ein Stoßwellensensor 15 enthalten. In der gezeigten Ausführungsform ist der Stoßwellensensor 15 ein elektrischer Sensor, speziell eine Piezokeramik oder ein Piezokristall 17, welcher über eine Zuleitung 19 an ein Meßgerät 21 angeschlossen ist. Der Piezokristall 17 ist vorzugsweise im Zentralbereich oder in der Mitte des Ankoppelkörpers 11, d. h. auf der Zentralachse 22 der Stoßwellenquelle 1, angeordnet. Es ist auch möglich, mehrere Piezokristalle 17 nebeneinander vorzusehen, und zwar in radialer Richtung bezüglich der Zentralachse 22 oder auf einem Ring um die Zentralachse 22 herum.

Im normalen Betrieb der Stoßwellenquelle 1, also beispielsweise während der Lithotripsiebehandlung des Patienten 13, kann mit Hilfe des Piezokristalls 17 und des Meßgeräts 21 fortlaufend die Funktion der Stoßwellenquelle 1 überprüft werden. Die Überprüfung besteht beispielsweise in der Überwachung der korrekten (d. h. vorgegebenen) Druckamplitude des Stoßwellenimpulses am Ort des Stoßwellensensors 15. Durch eine werkseitig zuvor vorgenommene Referenzmessung sei es z. B. bekannt, daß bei vorgegebenen Betriebsparametern von beispielsweise einer Betriebsspannung von 15 kV, einer Kondensatorkapazität von 0,5 μF, einer Vorlaufstreckenlänge von 20 cm, usw. am Ort des Stoßwellensensors 15 bei ordnungsgemäßem Betrieb und ordnungsgemäßer Positionierung eine vorgegebene Amplitude des Stoßwellenimpulses (Referenzwert) auf treten muß. Weicht während der fortlaufenden Therapiebehandlung, die bis zu 1000 Stoßwellenimpulse pro Patient umfassen kann, die vom Stoßwellensensor 15 ermittelte Druckamplitude um einen vorgegebenen Prozentsatz von dem Referenzwert ab, so lassen sich daraus Rückschlüsse auf mögliche Störungen in der Stoßwellenquelle 1 ziehen. Die Feststellung einer überhöhten Druckamplitude kann dann benutzt werden, die Therapiebehandlung zu unterbrechen, und eine zu kleine Druckamplitude kann ebenfalls Anlaß zur Unterbrechung der Therapiemaßnahme und darauffolgende Überprüfung der Anlage geben.

Darüber hinaus kann mit dem in den Ankoppelkörper 11 eingebauten Stoßwellensensor 15 die Stoßwellenquelle 1 nach einer eventuellen Reparatur oder Wartung neu geeicht oder eingestellt werden. Wurde beispielsweise als Stoßwellengenerator 3 bekanntermaßen eine elektromagnetische Flach-Spule eingesetzt, die bei der Wartung gegen eine andere Spule ausgetauscht wurde, so besteht die Möglichkeit, daß das Zentrum der neuen Spule geringfügig verschoben ist. Demzufolge wird bei Auftreffen eines Stoßwellenimpulses am Stoßwellensensor 15 nicht der erwartete Referenzwert auftreten, sondern ein geringerer Wert. Die Spule kann so lange neu justiert werden, bis der vorgegebene Referenzwert erreicht wird. Dann ist sichergestellt, daß die Stoßwellenquelle 1 die gleichen Eigenschaften aufweist wie vor der Wartung oder Reparatur.

Der Referenzwert, der zur Neueinstellung der Stoßwellenquelle 1 herangezogen wird, braucht nicht der gleiche zu sein wie im sogenannten "On-Line"-Betrieb mit dem Patienten 13. So beispielsweise kann der Betriebsparameter Spannungswert nur 12 kV anstelle von den erwähnten 15 kV bei der Therapiebehandlung betragen oder einen Bereich von z. B. 12 kV bis 20 kV durchfahren.

In Fig. 2 sind gleiche Teile mit gleichen Bezugszeichen versehen wie in Fig. 1.

Die Stoßwellenquelle 1 besteht wiederum aus einem Stoßwellengenerator 3, einer Vorlaufstrecke 5 mit zugehörigem Fokussierungsmittel und einer Auskoppelmembran 7. In einer außenliegenden Halterung 9 ist randseitig eine perforierte Metallmembran 30 als Stabilisator eingespannt. Die Metallmembran 30 besitzt in ihrem Zentrum eine Aussparung 32, welche koaxial zur Zentrumsachse 22 der Stoßwellenquelle 1 verläuft. Die Aussparung 32 ist mit einer teilweise piezoelektrischen Folie, beispielsweise einer PVDF-Folie 34 überspannt, die in ihrem zentralen Bereich piezoelektrisch aktiviert, also polarisiert ist. Auf beiden Seiten der PVDF-Folie 34 ist je eine ringförmige Ableitelektrode 36 vorgesehen, welche außerhalb der polarisierten Fläche angeordnet ist. Die Ableitelektroden 36 sind an Leitungen 19 angeschlossen, welche zu einem Meßgerät 21 führen. Die PVDF-Folie 34 und die ringförmigen Ableitelektroden 36 bilden in diesem Ausführungsbeispiel einen Stoßwellensensor 15. Ein solcher Stoßwellensensor 15 ist ausführlich in der deutschen Patentanmeldung P 35 45 382.6 beschrieben, deren Inhalt zur Offenbarung vorliegender Beschreibung gehören soll.

Der Stoßwellensensor 15 ist in einem formstabilen, gelartigen Ankoppelkörper 11 eingebettet, welcher durch die perforierte Metallmembran 30 gehalten wird. Der scheibenförmig ausgebildete Ankoppelkörper 11 liegt mit feuchtgehaltener Auflagefläche luftblasenfrei an der Auskoppelmembran 7 an. Auf der anderen (ebenfalls feucht gehaltenen) Auflagefläche wird der zu behandelnde Patient angekoppelt.

Das Auftreffen eines Stoßwellenimpulses auf die PVDF-Folie 34 wird durch kapazitive Messung über die Ableitelektroden 36 vom Meßgerät 21 ermittelt. Aus dem Meßwert kön nen Rückschlüsse auf die Amplitude des Stoßwellenimpulses gezogen werden. Funktionsweise und Handhabung des Ankoppelkörpers 11 sind identisch zu denen gemäß der Ausführungsform nach Fig. 1. Auch hier ist ein "On-Line"-Betrieb, also eine fortlaufende Überwachung der Stoßwellenimpulse während der Therapiebehandlung, möglich.

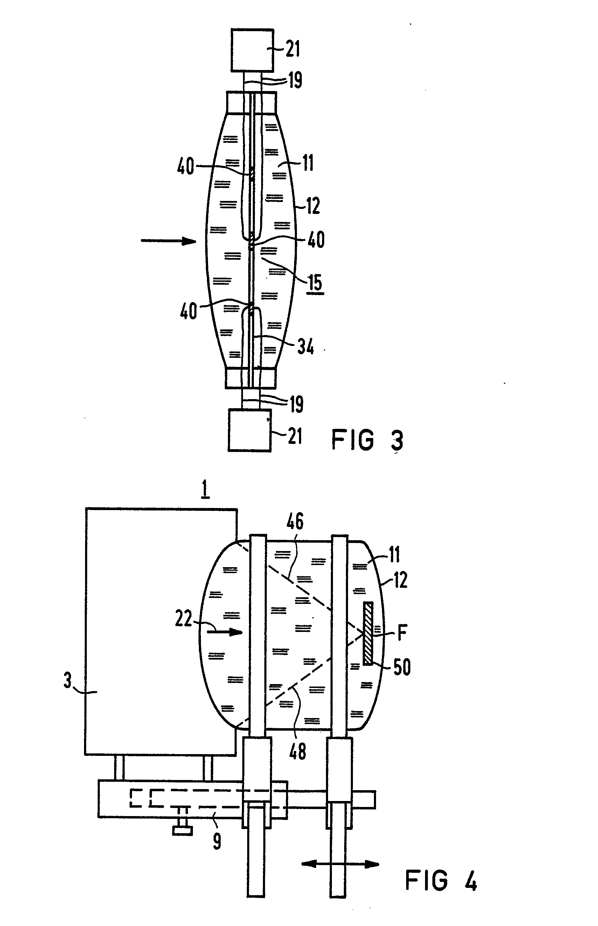

In Fig. 3 ist ein Ankoppelkörper 11 mit integriertem Stoßwellensensor 15 allein dargestellt. Der Stoßwellensensor umfaßt hier eine großflächige, mit Aussparungen versehene PVDF-Folie 34, auf welcher an vorgegebenen kleinen Teilflächen je eine Polarisation (d. h. piezoelektrische Aktivierung) vorgenommen und ein Metallkontakt 40 aufgedampft ist. Die Metallkontakte 40 sind jeweils über eine Leitung 19 mit einem Meßgerät 21 verbunden. Gemäß dieser Ausführungsform wird die Ladung, die auf der von einem Stoßwellenimpuls getroffenen aktivierten Sensorfläche entsteht, galvanisch mit Hilfe der Metallkontakte 40 detektiert, galvanisch durch die Leitungen 19 weitergegeben und in dem zugehörigen Meßgerät 21 zu je einem Meßwert, z. B. einem den zeitlichen Druckverlauf wiedergebenden Spannungssignal, verarbeitet. Bei dieser Ausführungsform sind mehrere nebeneinander oder auch vor-und hintereinanderliegende Meßstellen bei Einsatz mehrerer PVDF-Folien gleichzeitig möglich.

In Fig. 4 ist eine Ausführungsform dargestellt, wie sie typischerweise bei Montage-, Prüf-oder Wartungsarbeiten zur Anwendung kommt. Ein gelartiger, formstabiler Ankoppelkörper 11 ist als "Klotz" ausgebildet; d. h. daß er solche Ausmaße aufweist, daß der Fokus F einer Stoßwellenquelle 1 noch innerhalb des Ankoppelkörpers 11 liegt. Dies ist durch die Randstrahlen 46, 48 angedeutet. Vorliegend liegt die Fokusebene nahe der patientenseitigen Ankoppelfläche 12. Am Ort des erwarteten Fokus F, also nahe der Ankoppelfläche 12, ist ein dünner, ebener Stoßwellenindikator 50 symmetrisch zu der Zentralachse 22 in den Ankoppelkörper 11 eingebracht. Der Stoßwellenindikator 50 besteht beispielsweise aus einem runden Keramikplättchen, das unter der Einwirkung von Stoßwellen eine Materialabtragung erfährt, oder einer dünnen Metall-, speziell Bleifolie, die sich unter Einwirkung von Stoßwellen verformt oder ausbeult. Elektrische Verbindungsleitungen sind hier also nicht erforderlich. Nach den erwähnten Arbeiten wird der Ankoppelkörper 11 so in einer Halterung 9 positioniert, daß die werksseitig vorgegebenen Einstellwerte für den Abstand zum Stoßwellengenerator 3, für die Größe der Vorlaufstrecke 5, für den Abstand zum Fokussierungsmittel, usw. erfüllt sind. Nach Auslösung einer oder mehrerer Stoßwellenimpulse kann das Betreiberpersonal an dem Stoßwellenindikator 50, und zwar durch Sichtkontrolle am Indikator 50 in der Fokusebene, feststellen, ob eine mechanische Verformung oder Abtragung an der gewünschten Fokusstelle vorliegt, d. h. ob der Fokus F tatsächlich an der vorausbestimmten Stelle liegt. Für den Fall, daß dies nicht zutrifft, sind weitere Prüf-und Justierarbeiten vorzunehmen.

Der Stoßwellenindikator 50 ist vorzugsweise mit einer Markierung versehen, die ähnlich einer (Wurf-oder Schieß-) Zielscheibe mit Sektoren und Kreisringen versehen ist. Dadurch können Abweichungen der Fokuslage quantitativ erfaßt werden, was den Aufwand bei der nachfolgenden Justierung reduziert.

## Ansprüche

1. Ankoppelkörper für die Ankopplung einer Stoßwellenquelle, insbesondere für die Übertragung von Stoßwellen von einer Stoßwellenquelle zu einem zu behandelnden Patienten, wobei der Ankoppelkörper aus einem elastischen, formstabilen Material mit feuchten Außenflächen gebildet ist, **dadurch gekennzeichnet, daß** im elastischen, formstabilen Material ein Stoßwellensensor (15, 50) enthalten ist.

2. Ankoppelkörper nach Anspruch 1, **dadurch gekennzeichnet, daß** der Stoßwellensensor (15) eine piezoelektrische PVDF-Folie (34) umfaßt (Fig. 2, 3).

3. Ankoppelkörper nach Anspruch 1, **dadurch gekennzeichnet, daß** der Stoßwellensensor einen Piezokristall (17) oder eine Piezokeramik umfaßt (Fig. 1).

4. Ankoppelkörper nach Anspruch 1, **dadurch gekennzeichnet, daß** der Stoßwellensensor (15) eine dünne Metallfolie (50) umfaßt, die unter Einwirkung von Stoßwellen verformbar ist (Fig. 4).

5. Ankoppelkörper nach Anspruch 1, **dadurch gekennzeichnet, daß** der Stoßwellensensor (15) ein Keramikplättchen ist, das unter Einwirkung von Stoßwellen abgetragen wird.

6. Ankoppelkörper nach einem der Ansprüche 1 bis 5, **dadurch gekennzeichnet, daß** er - scheibenförmig ausgebildet und der Stoßwellensensor (15) mittig, insbesondere zentral, angeordnet ist.

7. Ankoppelkörper nach einem der Ansprüche 1 bis 5, **dadurch gekennzeichnet, daß** er - scheibenförmig ausgebildet und eine Stirnfläche aufweist, an der der Stoßwellensensor (15) angeordnet ist.

8. Ankoppelkörper nach einem der Ansprüche 1 bis 7, **dadurch gekennzeichnet, daß** er aus einem Hydrogel besteht.

9. Ankoppelkörper nach einem der Ansprüche 1 bis 8, **dadurch gekennzeichnet, daß** er einen wesentlich größeren Durchmesser besitzt als der Stoßwellensensor (15).

10. Ankoppelkörper nach einem der Ansprüche 1 bis 9, **dadurch gekennzeichnet,** daß der Stoßwellensensor (15; 50) nahe einer Ankoppelfläche (12) angeordnet ist (Fig. 4).

FIG 1

FIG 2

FIG 3

FIG 4